# FASCICULE DE BREVET EUROPEEN

(11) **EP 0 656 055 B1**
(45) Date de publication et mention de la délivrance du brevet: **09.10.2002**
(21) Numéro de dépôt: 93917855.4
(22) Date de dépôt: 05.08.1993
(51) Int. Cl.: C12N 7/06

(54) **PROCEDE D'INACTIVATION VIRALE DES PRODUITS PLASMATIQUES PAR DES FLUIDES SUPERCRITIQUES OU SUBCRITIQUES**
VERFAHREN ZUR INAKTIVIERUNG VON VIRUS IN PLASMAPRODUKTE DURCH ÜBER- ODER UNTERKRITISCHEN FLÜSSIGKEITEN
METHOD FOR THE VIRAL INACTIVATION OF PLASMATIC PRODUCTS BY USING SUPERCRITICAL OR SUBCRITICAL FLUIDS

(30) Priorité: 06.08.1992 FR 9209767
(43) Date de publication de la demande: 07.06.1995
(73) Titulaire: ASSOCIATION POUR L'ESSOR DE LA TRANSFUSION SANGUINE DANS LA REGION DU NORD, 59000 Lille (FR)
(72) Inventeur: BURNOUF, Thierry, F-59136 Wavrin (FR); BURNOUF, Miryana, F-59136 Wavrin (FR); BOUZIDI, Ahmed, F-59112 Annoeuillin (FR); PERRUT, Michel, F-69100 Villeurbanne (FR)
(74) Mandataire: Lhuillier, René
(86) Numéro de dépôt international: FR9300795
(87) Numéro de publication internationale: WO94003590

(56) Documents cités:
- EP-A- 0 322 687
- WO-A-87/02697
- PTS NEWSLETTER DATABASE, PREDICASTS, CLEVELAND, ABSTRACT NO. 01504003, "Low-Temperature Viral Inactivation R&D" & Antiviral Agents Bulletin, vol. 5, no. 5, mai 1992

## Description

La présente invention concerne, de façon générale, l'application des fluides supercritiques ou subcritiques au traitement des "produits plasmatiques" c'est-à-dire du plasma total, et généralement de tous produits ou substances extraits ou dérivés du plasma provenant du sang humain ou animal, et notamment le surnageant de cryoprécipité, les fractions plasmatiques, le sérum.

Plus précisément, elle a pour objet un nouveau procédé d'inactivation des agents viraux que ces produits plasmatiques contiennent, par mise en contact avec un fluide constitué par un corps pur ou un mélange de corps purs en état supercritique ou subcritique.

En variante, l'invention a également pour objet un procédé d'inactivation virale et de délipidation des produits plasmatiques, par mise en contact desdits produits plasmatiques avec un fluide présentant un caractère lipophile et constitué par un corps pur ou un mélange de corps purs en état supercritique ou subcritique.

En particulier, ont été étudiés le dioxyde de carbone (CO₂) et le protoxyde d'azote (N₂O) à l'état de fluides supercritiques ou subcritiques. Ces deux corps présentent une absence de toxicité déjà largement démontrée, mais surtout paraissent aptes à ne provoquer qu'un minimum de dénaturation des protéines présentes dans les produits plasmatiques.

En effet Demanderesse a pu constater que, malgré des conditions de mise en oeuvre relativement sévères, lesquelles sont nécessaires pour obtenir l'état supercritique ou subcritique des fluides retenus, l'ensemble des protéines présentes dans le plasma humain, dont la fragilité est reconnue, est néanmoins préservé de manière très satisfaisante puisque plus de 80 % de l'ensemble de l'activité biologique est maintenu, ce fait s'appliquant particulièrement aux protéines labiles d'extrême importance thérapeutique que sont certains facteurs de coagulation sanguine.

La mise en oeuvre du traitement selon l'invention permet d'inactiver des virus de tous types, c'est-à-dire enveloppés ou non, à ADN ou à ARN. Des virus marqueurs de différents types ont été utilisés comme modèles expérimentaux pour démontrer l'efficacité du traitement selon l'invention et ont été délibérément additionnés (jusqu'à des titres de 4-7 log₁₀) dans les produits plasmatiques afin de mesurer leur taux d'inactivation en fin de traitement.

La courbe de vaporisation (changement d'état liquide-gaz) d'un fluide pur présente un point d'arrêt dit "point critique" qui correspond à un couple de pression-température (P_{c}, T_{c}) assez facile à atteindre pour la plupart des composés. Au-delà de ce point critique (P>P_{c} et T>T_{c}), un seul état existe : le fluide est alors dit "supercritique".

L'état-supercritique est caractérisé
- soit par une pression et une température supérieures à la pression et à la température critique du fluide s'il s'agit d'un corps pur,
- soit, pour un mélange de composition fixée de deux ou plusieurs constituants miscibles dans ces conditions, par un point représentatif (pression, température) situé à l'extérieur de l'enveloppe de la zone diphasique sur un diagramme (pression, température) et correspondant à une pression et une température supérieures à la pression et à la température du point critique du mélange, point critique défini comme le point de raccordement des courbes de rosée et de bulle dudit mélange.

L'état subcritique est caractérisé
- soit par une pression supérieure à la pression critique et une température inférieure à la température critique du fluide s'il s'agit d'un corps pur,
- soit, pour un mélange de composition fixée de deux ou plusieurs constituants miscibles dans ces conditions, par un point représentatif (pression, température) situé à l'extérieur de l'enveloppe de la zone diphasique sur un diagramme (pression, température) et correspondant à une pression supérieure à la pression du point critique du mélange et à une température inférieure à la température du point critique du mélange, point critique défini comme le point de raccordement des courbes de rosée et de bulle dudit mélange.

Les fluides en état supercritique ou subcritique ont des propriétés physicochimiques particulières.

Considérant un même corps ou mélange, ceux-ci présentent une masse spécifique équivalente à celle du liquide, une viscosité faible se rapprochant de celle du gaz, un coefficient de diffusion au moins dix fois plus élevé que le liquide et, dans les échanges de matière, un coefficient de transfert très important. Enfin ils peuvent être aisément séparés du milieu où ils ont été utilisés de manière totale par détente.

On a déjà proposé d'utiliser les fluides en état supercritique ou subcritique, notamment le dioxyde de carbone (CO₂), le protoxyde d'azote (N₂O), des hydrocarbures "légers" (en C₂ à C₅), des hydrocarbures halogénés et d'autres encore, dans des applications aussi variées que l'extraction fluide-solide, par exemple pour décaféiner le café ou le thé, le fractionnement fluide-liquide, la chromatographie préparative, certaines réactions chimiques ou biochimiques, l'élaboration et le traitement de certains matériaux.

Un exposé général de ces applications est fait dans un article de M. PERRUT, paru dans Informations Chimie n° 321, octobre 1990, pages 166-177 qui cite un grand nombre de références. On peut également citer l'ouvrage "Proceedings of the 1st International Symposium on Supercritical Fluids, Nice (17-19 octobre 1988), M. PERRUT, ed., Librairie Lavoisier, Paris ; l'article du même auteur dans Biofutur, juin 1989, pages 43-47, en ce qui concerne l'extraction par les fluides supercritiques et "LE TECHNOSCOPE DE BIOFUTUR", n° 25, janvier 1989 rédigé par C. BERGER et M. PERRUT, en ce qui concerne la "Purification de molécules d'intérêt biologique par chromatographie préparative avec éluant supercritique".

Aucun de ces documents ne propose, ni même ne suggère, l'application des fluides supercritiques ou subcritiques à l'inactivation virale des produits plasmatiques.

De nombreux dérivés sont purifiés à partir du plasma sanguin humain à des fins thérapeutiques. Il s'agit en particulier des facteurs de la coagulation sanguine (Facteur VIII, facteur von Willebrand, Facteur IX etc...), de l'albumine, de l'α₁-antitrypsine, des immunoglobulines, du fibrinogène etc... Le principal problème posé par l'utilisation de produits dérivés du sang humain est le risque de contamination par un ou plusieurs virus résiduels mal inactivés.

Des procédés d'inactivation virale par pasteurisation sont couramment utilisés, toutefois ils nécessitent souvent la mise au point de formules de stabilisants complexes pour préserver l'activité biologique de certaines molécules particulièrement fragiles. Certains virus modèles particulièrement résistants sont partiellement inactivés par ce traitement. D'autres techniques de chauffage (chaleur à sec, par exemple) présentent une efficacité inférieure à celle de la pasteurisation.

Un autre traitement dont l'efficacité a été bien démontrée pour l'inactivation virale est le procédé utilisant un traitement au solvant-détergent (brevets US 4 314 977 et 4 315 919). Ce traitement permet en général le maintien de l'activité biologique des protéines à purifier mais il nécessite une parfaite élimination des réactifs utilisés, ce qui entraîne la mise en oeuvre de traitements relativement complexes, généralement basés sur des étapes de chromatographie. Il n'est, par ailleurs, efficace que sur les virus enveloppés.

L'invention a pour but de remédier aux inconvénients des procédés de décontamination connus des produits plasmatiques ou de servir de complément à ces procédés grâce à un traitement permettant une inactivation des contaminants viraux, tout en préservant l'activité des molécules d'intérêt thérapeutique et qui, sans nécessiter d'étapes complexes d'élimination de l'agent décontaminant, en assure l'élimination totale.

Selon l'invention, il a été trouvé qu'il était possible d'inactiver des virus pathogènes des produits plasmatiques, tout en maintenant intactes de manière surprenante et inattendue, les propriétés biologiques des molécules d'intérêt thérapeutique, en particulier les protéines qui y sont contenues, en soumettant ces produits plasmatiques à un traitement par un fluide supercritique ou subcritique, de nature compatible avec lesdites molécules et dans des conditions de température, de pression et de pH également compatibles avec lesdites molécules.

La présente invention a donc essentiellement pour objet l'application des fluides supercritiques ou subcritiques, tels que définis dans ce qui précède, à l'inactivation virale des produits plasmatiques selon la définition donnée plus haut.

Il va de soi que le corps ou le mélange de corps utilisé sous forme de fluide supercritique ou subcritique selon l'invention ne doit pas dénaturer les molécules d'intérêt thérapeutique contenues dans les produits plasmatiques, telles que notamment certaines protéines. De plus, il doit présenter une pression critique P_{c} et surtout une température critique T_{c} telles que les pressions et températures pour lesquelles il se trouve à l'état supercritique ou subcritique soient compatibles avec ces molécules.

De même, le pH du milieu de traitement doit pouvoir être maintenu au voisinage d'une valeur ne portant pas atteinte à ces mêmes molécules d'intérêt thérapeutique.

Enfin, le contact entre les produits plasmatiques et le fluide utilisé doit être suffisamment intime et long pour assurer l'efficacité du processus d'inactivation des virus pathogènes qui y sont contenus.

Des corps non dénaturants, au sens de l'invention, qui peuvent être utilisés sont notamment les hydrocarbures légers en C₂ à C₈ et particulièrement en C₂ à C₆, tels l'éthane (T_{c} = 32,3°C, P_{c} = 4,88 MPa) et l'éthylène (T_{c} = 9,3°C, P_{c} = 5,04 MPa), le dioxyde de carbone (T_{c} = 31°C, P_{c} = 7,38 MPa), le protoxyde d'azote (T_{c} = 36,5°C, P_{c} = 7,24 MPa).

On peut aussi considérer les hydrocarbures halogénés, avec toutefois les restrictions dont leur usage fait l'objet par voie de réglementations spécifiques encore très incomplètement définies au jour de la présente demande de brevet. Il s'agit par exemple du trifluorométhane, CHF₃ (T_{c} = 26,2°C, P_{c} = 4,86 MPa), du monofluorométhane, CH₃F (T_{c} = 4,9°C, P_{c} = 5,6 MPa).

Les mélanges de corps peuvent également être utilisés, en toutes proportions. Des proportions où l'un des corps en mélange est présent à raison d'environ 10 % ou plus introduisent une modulation notable des constantes critiques du mélange dans le sens d'une évolution vers les valeurs caractéristiques de ce corps. Des proportions où l'un des corps en mélange est présent à raison d'environ 1 % à 5 % n'introduisent en général qu'une modulation négligeable à très faible des constantes critiques du mélange. Ces mélanges de corps peuvent être constitués des corps déjà définis ci-dessus, ou peuvent comporter en proportions variables des corps qui, pris individuellement, n'auraient pu faire l'objet d'une mise en oeuvre de l'invention à cause de leurs caractéristiques intrinsèques et notamment leurs constantes critiques, mais qui, présents dans un mélange, apportent une propriété particulièrement intéressante dans le cadre de la mise en oeuvre du procédé selon l'invention. Dans cette dernière catégorie on peut notamment citer certains hydrocarbures tels ceux en C₉ ou plus, les composés organiques oxygénés et plus particulièrement les alcools et les acides, les composés organiques azotés et plus particulièrement les amines, les acides et les bases d'une manière générale.

Le ou les corps utilisés présentent avantageusement un caractère lipophile. Dans ce cas, la mise en oeuvre du procédé selon l'invention conduit non seulement à une inactivation virale du produit plasmatique traité, mais également à sa délipidation au moins partielle, ce qui contribue notamment à améliorer sa tolérance lors de son utilisation thérapeutique.

Le dioxyde de carbone et le protoxyde d'azote répondant aux critères principaux de choix ont été plus spécialement étudiés parce qu'ils présentent en plus :
- une température critique inférieure à et voisine de la température normale du corps humain,
- une pression critique modérée,
- un caractère lipophile apte à améliorer la tolérance aux produits thérapeutiques issus du traitement,
ces qualités laissant espérer un minimum d'altération des molécules des produits plasmatiques, et une amélioration de certaines de leurs qualités thérapeutiques, et
- une absence de toxicité,
- un caractère non inflammable,
- une grande disponibilité,
précieux atouts pour une production industrielle de produits à usage thérapeutique.

Il a en effet été démontré, par la mise en application du mode de réalisation préféré de la présente invention, que des produits plasmatiques soumis à l'action du dioxyde de carbone ou du protoxyde d'azote, en état supercritique ou subcritique, voyaient l'activité biologique de leur contenu en protéines destinées à des fins thérapeutiques préservée. La dénaturation de ces molécules pouvant résulter de réactions chimiques ou d'effets physiques, et notamment calorifiques, consécutifs au traitement par ces corps en état supercritique ou subcritique est très limitée. Cette dénaturation très limitée mais qui pourrait être plus importante à des températures supérieures à 40°C, peut être réduite par l'addition de stabilisants tels certains sucres, polyols ou acides aminés.

Il a ainsi pu être démontré la conservation de l'activité biologique de protéines labiles du plasma, tels les fragiles facteurs de coagulation sanguine, à plus de 80 %, après traitement par le dioxyde de carbone ou le protoxyde d'azote en état supercritique, avec des résultats satisfaisants d'inactivation virale et de délipidation du plasma.

Selon un mode préféré de réalisation, le procédé de traitement selon l'invention, défini ci-dessus, est mis en oeuvre en utilisant le dioxyde de carbone ou le protoxyde d'azote en état supercritique ou subcritique, à une température comprise entre 10 et 65°C, de préférence entre 30 et 65°C, et sous une pression comprise entre P_{c} et 10 P_{c}, et de préférence comprise entre Pc et 5 P_{c}.

La stabilisation des produits plasmatiques est souhaitable, comme décrit dans la présente invention, afin d'améliorer la conservation de l'activité biologique de leurs protéines.

Différents moyens sont possibles pour parvenir à cette stabilisation.

Ainsi il est souhaitable de maintenir le pH au voisinage de 7,5 par exemple en tamponnant le milieu à traiter avec des tampons appropriés comme le tris-HCl, un tampon phosphate ou un tampon citrate, ou en utilisant un mélange binaire de corps en état supercritique ou subcritique. Le pH peut encore être régulé et stabilisé par différents gaz comme l'oxygène. Cette maîtrise du pH est particulièrement nécessaire dans le cas de fluides modifiant le pH du milieu à traiter comme c'est le cas du dioxyde de carbone et de son action acidifiante.

Il peut en outre être avantageux de stabiliser le produit plasmatique traité par des agents stabilisants tels que des sucres, des polyols, ou des acides aminés, ou encore des combinaisons de ces composants. Parmi ces agents, on peut citer en particulier le saccharose, le glucose, le fructose, le sorbitol, le mannitol, le glycérol, le glycocolle, l'arginine, ou encore la lysine.

Dans certains cas, par exemple pour stabiliser certaines protéines comme les facteurs de coagulation sanguine, on peut utiliser aussi le chlorure de calcium, le gluconate de calcium et l'héparine.

Une stabilisation appropriée des protéines en question permet de travailler dans une gamme de température d'environ 5 à 80°C. Quel que soit le fluide utilisé, le procédé selon l'invention est mis en oeuvre à la température de traitement et à la pression opératoire choisies dans les gammes appropriées, pendant tout ou partie du traitement, à condition dans ce dernier cas, notamment de ne pas dépasser les températures et pressions compatibles avec le produit, éventuellement stabilisé, soumis au traitement.

Le contact entre le produit plasmatique d'une part, et le fluide supercritique ou subcritique ("fluide de traitement") d'autre part, peut être réalisé selon différentes techniques, en fonction des objectifs visés. La durée du contact dépend essentiellement des conditions de sa mise en oeuvre. Elle varie de 30 minutes à 12 heures.

Par ailleurs, pour augmenter l'efficacité du traitement ou pour des raisons de facilité de mise en oeuvre, le produit plasmatique peut être soumis à une étape préliminaire de mise en forme telle que, par exemple, une lyophilisation ou un cryobroyage.

Dans tous les cas, le corps, notamment le dioxyde de carbone ou le protoxyde d'azote, ou le mélange de corps devant être utilisé à l'état supercritique ou subcritique, est mis dans les conditions d'utilisation selon les procédés habituellement mis en oeuvre pour ce faire, à savoir par exemple :
- soit initialement liquéfié dans un réservoir, il est pompé à la pression requise, de préférence grâce à une pompe volumétrique doseuse à membrane, puis porté dans un échangeur à la température requise ;
- soit initialement à l'état gazeux, il est comprimé dans un compresseur, de préférence dans un compresseur "sec" à membrane, puis refroidi dans un échangeur à la température requise.

Lorsqu'il est dans les conditions de température et de pression requises pour l'utilisation, il est introduit dans un autoclave (ou tout autre récipient supportant des pressions élevées) où il est mis en contact avec le produit plasmatique à traiter. Ce contact peut être réalisé selon différents procédés, notamment :

### 1° - Par simple mise en contact statique des deux fluides.

Celle-ci est réalisée à la pression et à la température fixées, pendant un temps suffisamment long pour permettre une bonne diffusion du fluide de traitement au sein du produit plasmatique où il est partiellement dissous, notamment dans le cas du dioxyde de carbone ou du protoxyde d'azote. Cette opération est suivie d'une décompression de l'autoclave par lente évacuation du fluide de traitement qui peut être rejeté dans l'atmosphère, ou mieux encore recyclé.

On dispose utilement un second autoclave en aval de l'autoclave de traitement proprement dit afin de réaliser la captation de la partie du produit plasmatique susceptible d'être mécaniquement entraînée par le fluide de traitement lors de la phase de décompression-vidange.

Lorsque la pression dans l'autoclave est à nouveau voisine de la pression atmosphérique, on peut alors évacuer sans problème le produit plasmatique ainsi traité et réintroduire un second lot pour une nouvelle opération.

Cette technique présente l'avantage de ne pas nécessiter d'équipements de pompage des produits plasmatiques à haute pression et permet ainsi de limiter les altérations éventuelles de ceux-ci. Toutefois elle est lente car, d'une part, la surface de contact entre les deux fluides est très restreinte, et, d'autre part, la décompression jusqu'à la pression atmosphérique doit être conduite très lentement pour éviter l'entraînement mécanique d'une partie du produit plasmatique et le fort refroidissement de l'ensemble sous l'effet de la détente du fluide de traitement (effet Joule-Thomson).

### 2° - Par mise en contact dynamique des deux fluides par balayage continu par le fluide de traitement.

On peut ainsi remédier à certains inconvénients signalés ci-dessus. Le produit plasmatique est introduit, comme précédemment, dans l'autoclave, à la pression atmosphérique; le fluide de traitement, préalablement porté à la pression et à la température souhaitées, comme indiqué précédemment, est alors introduit en continu dans l'autoclave et en est extrait au même débit, de préférence à travers un second autoclave. Celui-ci permet de recueillir, par décantation, la partie de produit plasmatique qui pourrait être mécaniquement entraînée. Le fluide de traitement peut alors être soit rejeté dans l'atmosphère, soit, de préférence, recyclé après avoir été débarrassé des composés extraits dans l'autoclave de traitement, selon les techniques et avec les appareillages classiquement utilisés en extraction supercritique, c'est-à-dire décomprimé en plusieurs étapes assurant la séparation du fluide de traitement et des extraits, puis reconditionné selon l'une des procédures décrites ci-dessus (liquéfaction-pompage-réchauffage ou compression-refroidissement).

En fin d'opération, on arrête l'introduction du fluide de traitement et on décomprime lentement l'ensemble ainsi qu'il a été décrit dans la mise en oeuvre du contact statique.

Pour mettre en oeuvre cette opération, on utilise un autoclave qui se présente de préférence sous forme d'une colonne cylindrique verticale dont le rapport hauteur/diamètre est avantageusement supérieur à 10, de préférence de 20 à 50.

Le contact entre les deux fluides est favorisé par l'introduction du fluide de traitement en pied de colonne, à travers un distributeur constitué de préférence de matériau fritté et par l'installation d'un garnissage de corps creux (anneaux, par exemple) ou de plateaux perforés dans la colonne. Afin de limiter l'entraînement d'une partie du produit plasmatique par le fluide de traitement sortant de l'autoclave, on peut avantageusement utiliser des dispositifs classiques de dévésiculation comme des chicanes, des plaques perforées, un garnissage ou un matelas de fibres.

Une variante intéressante du mode de mise en contact consiste à utiliser le mécanisme classiquement connu et utilisé sous le nom de "gas-lift" pour mettre en mouvement le liquide à traiter et l'agiter fortement en assurant un contact intime avec le fluide de traitement, sans recourir à l'utilisation d'un agitateur mécanique ou à une pompe de circulation extérieure dont les mises en oeuvres sont très compliquées dans le cas d'une mise en contact avec un fluide sub- ou supercritique. On peut ainsi disposer dans un autoclave en forme de cylindre vertical, un autre cylindre coaxial d'un diamètre inférieur et alimenter le fluide de traitement soit uniquement dans la couronne extérieure comprise entre la paroi de l'autoclave et ledit cylindre intérieur, soit uniquement à l'intérieur dudit cylindre intérieur, ce qui va provoquer le mécanisme de "gas-lift" et l'agitation du liquide en contact intime avec le fluide de traitement.

Si elle présente l'avantage de ne pas nécessiter le pompage à haute pression du produit plasmatique, cette méthode de mise en contact dynamique est lente à mettre en oeuvre, compte tenu du temps nécessaire pour la décompression à la fin de la mise en contact de chaque échantillon.

### 3° - Par mise en contact continu des deux fluides circulant à contre-courant.

On peut ainsi remédier à certains inconvénients mentionnés ci-dessus.

Pour cela, on utilise une technique et un équipement analogues à ceux utilisés classiquement lors d'opérations d'extraction/fractionnement par un fluide à l'état supercritique sur des charges liquides, ainsi qu'il a été plusieurs fois décrit dans l'art antérieur.

Le contacteur est constitué d'une colonne cylindrique verticale au sein de laquelle les deux fluides circulent à contre-courant :
- le produit plasmatique pompé à la pression de travail, de préférence grâce à une pompe volumétrique doseuse à membrane, est introduit au sommet de la colonne ;
- le fluide de traitement à l'état supercritique ou subcritique, conditionné comme décrit précédemment, est introduit en pied de colonne, de préférence grâce à un distributeur constitué de matériau fritté.

Le contact entre les deux phases est favorisé de façon connue par l'installation de plateaux perforés ou de corps de garnissage au sein de la colonne. On peut soit réaliser ce contact en état dit "noyé" si on maintient le niveau du produit plasmatique jusqu'au sommet de la colonne, le fluide de traitement étant alors la phase dispersée, ou en état dit "arrosé" si on maintient le niveau du produit plasmatique en pied de colonne, le fluide de traitement étant alors la phase continue.

La régulation de ce niveau au sommet ou en bas de la colonne, selon le choix de l'opérateur, permet l'évacuation continue ou séquentielle du produit plasmatique traité, par le pied de la colonne, vers plusieurs autoclaves disposés en série où sera réalisée la décompression du liquide recueilli jusqu'à la pression atmosphérique, avec évacuation progressive à l'état gazeux du fluide de traitement qui s'y était dissous à haute pression, lors du contact dans la colonne.

Par ailleurs, le fluide de traitement est évacué en tête de colonne, après percolation éventuelle à travers un dispositif classique de dévésiculation, par une vanne pilotée par la régulation de la pression dans la colonne, vers un ensemble de décompression au sein duquel la pression est graduellement diminuée et les composés extraits sont séparés, avant évacuation dans l'atmosphère ou de préférence recyclage de ce fluide de traitement détendu, comme décrit ci-dessus (2°), dans les conditions décrites dans l'art antérieur.

Cette mise en oeuvre permet le traitement de grands volumes de produits plasmatiques.

### 4° - Par mise en contact d'une matière solide avec le fluide de traitement.

On peut ainsi traiter des produits plasmatiques se présentant sous forme solide, et en particulier ceux obtenus après un processus de lyophilisation.

Pour cela on utilise une technique et un appareillage décrits dans l'art antérieur afin de réaliser des opérations d'extraction par un fluide à l'état supercritique sur des charges solides.

Il va de soi que compte tenu de la nature même des matières traitées et de leur destination (usage thérapeutique), les équipements et le fluide supercritique ou subcritique utilisés, ainsi que les conditions de traitement adoptées, doivent préserver l'activité biologique des molécules d'intérêt thérapeutique présentes, notamment des protéines. De plus, la stérilisation et l'aseptisation de l'appareillage et des produits utilisés doivent être parfaitement assurées avant la mise en oeuvre du procédé.

De même, la thermostatation de tous les équipements doit être précise, afin que leur température ne dépasse pas les températures préalablement fixées.

Pour cette raison, il est préférable d'utiliser le procédé dit "basse température" (condensation/pompage/réchauffage), pour conditionner et/ou recycler le fluide de traitement.

Pour la mise en oeuvre du procédé selon l'invention et afin notamment de préserver l'activité biologique des molécules d'intérêt thérapeutique présentes dans les fractions traitées, on utilise avantageusement des équipements en titane, ou des équipements en acier présentant une composition et des caractéristiques mécaniques et physicochimiques compatibles avec le traitement envisagé, tels que notamment certains aciers dits inoxydables, lesdits équipements étant chemisés avec des matériaux métalliques compatibles avec les molécules biologiques tels que le titane par exemple, ou avec des matériaux polymériques tels que le polytétrafluoroéthylène (PTFE).

Comme il a été indiqué plus haut, le procédé selon l'invention, en particulier lorsque le fluide supercritique ou subcritique utilisé est constitué de dioxyde de carbone ou de protoxyde d'azote, permet de façon surprenante et inattendue de décontaminer, par inactivation des virus pathogènes qu'ils contiennent, les produits plasmatiques et en particulier, les fractions plasmatiques obtenues par ailleurs par les méthodes classiquement utilisées dans les installations de fractionnement du sang.

En complément de ce résultat fondamental du procédé selon l'invention, certains fluides supercritiques ou subcritiques utilisés, notamment le dioxyde de carbone et le protoxyde d'azote, permettent, en raison de leur caractère lipophile, de délipider les produits plasmatiques et notamment le plasma total, ce qui contribue à améliorer la tolérance lors de leur utilisation thérapeutique.

Enfin, il est rappelé un avantage essentiel du procédé selon l'invention, à savoir l'élimination totale et facile du fluide de traitement ayant réalisé l'inactivation des virus pathogènes, étape obligatoire de toute préparation de produits plasmatiques dont l'usage est thérapeutique.

Les exemples suivants illustrent la présente invention sans toutefois en limiter la portée.

### Exemple 1 - Mise en oeuvre du procédé

### Préparation des échantillons :

1- Les différentes manipulations sont effectuées sur un mélange ou pool de plasmas d'au moins 6 donneurs différents, collectés par plasmaphérèse sur machine de type Haemonetics.
2- Le pool de plasma est séparé en trois lots distincts :
   - Le premier lot sert de plasma de référence et ne contient aucun stabilisant.
   - Le deuxième lot est stabilisé par l'utilisation d'une première formule de stabilisants dénommée FS1, comprenant, par rapport au volume de plasma de départ :
      - héparine : 0,7 U/ml;
      - citrate tri-sodique : 1, 47 mg/ml (citrate de Na 5mM) ;
      - gluconate de calcium : 2,69 mg/ml (gluconate de Ca 6mM)
      - lysine : 5 mg/ml;
      - arginine : 5 mg/ml;
      - pH = 7,5.
   - Le troisième lot est stabilisé par l'utilisation d'une seconde formule de stabilisants dénommé FS2, comprenant, par rapport au volume de plasma de départ : 
- acide citrique : 1,95 mg/ml
(citrate 10mM) ;
- citrate tri-sodique : 0,21 mg/ml
      - gluconate de calcium : 1,79 mg/ml (gluconate de Ca 4mM) ;
      - arginine : 5 mg/ml ;
      - lysine : 5 mg/mln ;
      - sorbitol : 1,38 g/ml ;
      - saccharose : 0,55 g/ml ;
      - pH = 7,5.

   Ces stabilisants sont ajoutés sous forme de poudre ou sous forme liquide concentrée (solution-mère) et sont ensuite dilués dans le plasma afin d'obtenir la concentration souhaitée.
3- Chaque lot est ensuite divisé en deux sous-lots qui sont traités indépendamment par le protoxyde d'azote et le dioxyde de carbone, respectivement.

La codification de ces 6 sous-lots est présentée de la manière suivante :

| | |
|---|---|
| 911 MO 330 | Plasma non stabilisé traité en N₂O |
| 911 MO 331 | Plasma stabilisé avec FS1 traité par N₂O |
| 911 MO 332 | Plasma stabilisé avec FS2 traité par N₂O |
| 911 MO 340 | Plasma non stabilisé traité par CO₂ |
| 911 MO 341 | Plasma stabilisé avec FS1 traité par CO₂ |
| 911 MO 342 | Plasma stabilisé avec FS2 traité par CO₂ |

Le traitement des différents sous-lots de plasma par le protoxyde d'azote (N₂O) ou par le dioxyde de carbone (CO₂) en phase supercritique est effectué à température constante, fixée à 37°C. Le volume traité par échantillon est de 15 ml.

Chaque tube est traité individuellement de la manière suivante :
- décongélation : 5 minutes à 37°C;
- introduction de l'échantillon dans un extracteur (autoclave) de 200 ml thermostaté à 37°C;
- montée en pression du N₂O ou du CO₂ sur une durée de 15 minutes jusqu'à obtention de la pression de traitement;
- ouverture de la sortie en aval de l'extracteur et introduction du N₂O ou du CO₂ pendant la durée du traitement à raison de 3 ml/min selon la méthode de contact dynamique sans contre-courant ;
- à la fin du temps d'extraction par N₂O ou CO₂ supercritique, arrêt de la pompe et diminution progressive de la pression jusqu'à environ 1 MPa en 30 minutes;
- soutirage du plasma total en bas de l'extracteur et récupération du liquide entraîné lors de l'extraction dans un pot de rétention rempli de billes de verre, disposé en aval de l'extracteur;
- congélation immédiate dès le soutirage.

Les paramètres qui varient sont le temps de contact du fluide supercritique sur les plasmas (0,5 h, 1 h, 1,5 h) ainsi que la pression appliquée (150 bars, 200 bars, 250 bars).

### Tableaux récapitulatifs et codification des différentes manipulations:

### Numérotation des échantillons :

- 1.1 = prélèvement dans l'autoclave;
- 1.2 = prélèvement dans le pot de rétention;
- 1.T = témoin maintenu à 37°C durant le temps de manipulation.

1. Plasmas traités au protoxyde d'azote (N₂O) :

| N₂O Pression (bars) | Lots de plasma | | | | | | | | |
|---|---|---|---|---|---|---|---|---|---|
| | 911 MO 330 | | | 911 MO 331 | | | 911 MO 332 | | |
| | 0,5 | h 1 h | 1,5 h | 0,5 h | 1 h | 1,5 h | 0,5 h | 1 h | 1,5 h |
| | 1.1 | 2.1 | 3.1 | 10.1 | 11.1 | 12.1 | 19.1 | 20.1 | 21.1 |
| 150 | 1.2 | 2.2 | 3.2 | 10.2 | 11.2 | 12.2 | 19.2 | 20.2 | 21.2 |
| | 1.T | 2.T | 3.T | 10.T | 11.T | 12.T | 19.T | 20.T | 21.T |
| | 4.1 | 5.1 | 6.1 | 13.1 | 14.1 | 15.1. | 22.1 | 23.1 | 24.1 |
| 200 | 4.2 | 5.2 | 6.2 | 13.2 | 14.2 | 15.2 | 22.2 | 23.2 | 24.2 |
| | 4.T | 5.T | 6.T | 13.T | 14.T | 15.T | 22.T | 23.T | 24.T |
| | 7.1 | 8.1 | 9.1 | 16.1 | 17.1 | 18.1 | 25.1 | 26.1 | 27.1 |
| 250 | 7.2 | 8.2 | 9.2 | 16.2 | 17.2 | 18.2 | 25.2 | 26.2 | 27.2 |
| | 7.T | 8.T | 9.T | 16.T | 17.T | 18.T | 25.T | 26.T | 27.T |

2. Plasmas traités au dioxyde de carbone (CO₂) :

| CO₂ Pression (bars) | Lots de plasma | | | | | | | | |
|---|---|---|---|---|---|---|---|---|---|
| | 911 MO 340 | | | 911 MO 341 | | | 911 MO 342 | | |
| | 0,5 h | 1 h | 1,5 h | 0,5 h | 1 h | 1,5 h | 0,5 h | 1 h | 1,5 h |
| | 1.1 | 2.1 | 3.1 | 10.1 | 11.1 | 12.1 | 19.1 | 20.1 | 21.1 |
| 150 | 1.2 | 2.2 | 3.2 | 10.2 | 11.2 | 12.2 | 19.2 | 20.2 | 21.2 |
| | 1.T | 2.T | 3.T | 10.T | 11.T | 12.T | 19.T | 20.T | 21.T |
| | 4.1 | 5.1 | 6.1 | 13.1 | 14.1 | 15.1 | 22.1 | 23.1 | 24.1 |
| 200 | 4.2 | 5.2 | 6.2 | 13.2 | 14.2 | 15.2 | 22.2 | 23.2 | 24.2 |
| | 4.T | 5.T | 6.T | 13.T | 14.T | 15.T | 22.T | 23.t | 24.T |
| | 7.1 | 8.1 | 9.1 | 16.1 | 17.1 | 18.1 | 25.1 | 28.1 | 27.1 |
| 250 | 7.2 | 8.2 | 9.2 | 16.2 | 17.2 | 18.2 | 25.1 | 26.2 | 27.2 |
| | 7.T | 8.T | 9.T | 16.T | 17.T | 18.T | 25.T | 26.T | 27.T |

### Exemple 2 - Mise en évidence de l'efficacité de traitement.

- Détermination du rendement protéique total et de l'activité biologique de facteurs de coagulation, choisis pour leur fragilité [le facteur V (F V) ; le facteur VII (F VII) ; le facteur VIII (F VIII) ; le facteur IX (F IX) ; le facteur von Willebrand (vWF) (activité cofacteur de la ristocétine); le fibrinogène (coagulabilité)].
- Electrophorèse, sur membrane d'acétate de cellulose, effectuée afin de visualiser la distribution des protéines du plasma (albumine ; globulines alpha 1, alpha 2, beta, gamma).
- Pouvoir virucide du traitement évalué sur différents virus marqueurs représentatifs [virus de l'immunodéficience humaine de type 1 (VIH-1) ; Sindbis ; Parainfluenza type 3 ; Herpès d'Aujeszki ; virus de la vaccine].
- Activité délipidante et dénaturante du traitement étudiée sur deux catégories de lipides du plasma, le cholestérol et les triglycérides.
- Lipoprotéinogramme permettant aussi d'évaluer la distribution électrophorétique des lipides plasmatiques : VLDL (pré-β-lipoprotéines) ; LDL (β-lipoprotéines) ; HDL (α-lipoprotéines).

### I Etude de l'activité biologique des protéines après traitement au fluide supercritique

Les tableaux suivants montrent que la perte d'activité biologique des protéines plasmatiques induite par le traitement au protoxyde d'azote ou au dioxyde de carbone en phase supercritique est très limitée, principalement en présence des stabilisants contenus dans les plasmas 911 MO 331, 911 MO 332, 911 MO 341 et 911 MO 342.

### (1) Traitement avec le protoxyde d'azote :

Les rendements sont établis par rapport à un témoin spécifique à chaque condition de traitement (voir ci-dessus les tableaux récapitulatifs et les codifications des différentes manipulations).

Ce témoin est maintenu durant le temps de manipulation à 37°C sans qu'il ne soit soumis à la pression de traitement par le protoxyde d'azote ou le dioxyde de carbone.

| Numérotation | | Activité résiduelle des différents facteurs de coagulation (%) après traitement au N₂O supercritique en absence de stabilisant | | | | | |
|---|---|---|---|---|---|---|---|
| 911 MO 330 | | F V | F VII | F VIII | F IX | vWF | fibrinogène |
| 150 bars 0,5 h | 1.1 | 75 | 67 | 66 | 73 | 72 | 66 |
| 150 bars 1 h | 2.1 | 75 | 60 | 63 | 75 | 69 | 66 |
| 150 bars 1,5 h | 3.1 | 67 | 62 | 61 | 68 | 68 | 61 |
| 200 bars 0,5 h | 4.1 | 73 | 66 | 69 | 72 | 70 | 65 |
| 200 bars 1 h | 5.1 | 69 | 66 | 61 | 67 | 70 | 61 |
| 200 bars 1,5 h | 6.1 | 66 | 61 | 58 | 63 | 64 | 57 |
| 250 bars 0,5 h | 7.1 | 58 | 64 | 60 | 69 | 67 | 64 |
| 250 bars 1 h | 8.1 | 64 | 65 | 58 | 60 | 68 | 54 |
| 250 bars 1,5 h | 9.1 | 62 | 57 | 51 | 62 | 63 | 53 |

Les différentes expérimentations effectuées sur le plasma non stabilisé en présence de protoxyde d'azote, suivant les paramètres rappelés ci-dessus, montrent une perte d'activité allant de 25 (0,5 h, 150 bars) à 50 % (1,5 h, 250 bars) pour les facteurs de coagulation sanguine. Ce phénomène est dû principalement à la technique de contact par bullage sous pression ainsi qu'à la température appliquées sur les protéines labiles plasmatiques.

| Numérotation | | Activité résiduelle des différents facteurs de coagulation (%) après traitement au N₂O supercritique en présence de FS1 | | | | | |
|---|---|---|---|---|---|---|---|
| 911 MO 331 | | F V | F VII | F VIII | F IX | vWF | fibrinogène |
| 150 bars 0,5 h | 10.1 | 88 | 90 | 85 | 90 | 91 | 86 |
| 150 bars 1 h | 11.1 | 83 | 88 | 82 | 88 | 93 | 85 |
| 150 bars 1,5 h | 12.1 | 84 | 82 | 85 | 70 | 88 | 90 |
| 200 bars 0,5 h | 13.1 | 87 | 89 | 84 | 89 | 89 | 86 |
| 200 bars 1 h | 14.1 | 90 | 92 | 81 | 88 | 87 | 84 |
| 200 bars 1,5 h | 15.1 | 83 | 86 | 79 | 89 | 86 | 75 |
| 250 bars 0,5 h | 16.1 | 86 | 87 | 82 | 89 | 87 | 85 |
| 250 bars 1 h | 17.1 | 80 | 83 | 81 | 89 | 87 | 82 |
| 250 bars 1,5 h | 18.1 | 83 | 84 | 78 | 82 | 80 | 80 |

Les rendements observés sur les préparations plasmatiques stabilisées avec la première formule de stabilisant montrent une amélioration très nette par rapport aux préparations plasmatiques non stabilisées.

L'apport des constituants composant cette solution stabilisante évite les effets calorifiques induits par le traitement au protoxyde d'azote sur les facteurs de coagulation qui y sont très sensibles, préservant ainsi approximativement 80 % de leur activité biologique dans les conditions les plus drastiques (1,5 h; 250 bars).

Ces résultats démontrent que ce traitement par le N₂O n'induit pas d'effet dénaturant significatif sur les préparations plasmatiques stabilisées.

| Numérotation | | Activité résiduelle des différents facteurs de coagulation (%) après traitement au N₂O supercritique en présence de FS2 | | | | | |
|---|---|---|---|---|---|---|---|
| 911 MO 332 | | F V | F VII | VIII | F IX | vWF | fibrinogène |
| 150 bars 0,5 h | 19.1 | 100 | 92 | 89 | 92 | 100 | 89 |
| 150 bars 1 h | 20.1 | 89 | 95 | 87 | 94 | 88 | 87 |
| 150 bars 1,5 h | 21.1 | 86 | 88 | 93 | 87 | 100 | 98 |
| 200 bars 0,5 h | 22.1 | 90 | 91 | 100 | 95 | 90 | 87 |
| 200 bars 0 h | 23.1 | 87 | 93 | 86 | 88 | 90 | 84 |
| 200 bars 1,5 h | 24.1 | 84 | 85 | 85 | 86 | 92 | 82 |
| 250 bars 0,5 h | 25.1 | 88 | 92 | 87 | 90 | 95 | 87 |
| 250 bars 1 h | 26.1 | 86 | 87 | 84 | 88 | 100 | 88 |
| 250 bars 1,5 h | 27.1 | 84 | 70 | 83 | 86 | 90 | 87 |

La perte minime d'activité biologique de protéines fragiles (moins de 20 % en moyenne pour l'ensemble des facteurs de coagulation dans les conditions les plus défavorables) dans les plasmas stabilisés avec la seconde formule démontre que le traitement au protoxyde d'azote supercritique n'induit qu'une altération physicochimique très limitée des protéines plasmatiques et ce indépendamment des pressions et des temps de contact appliqués lors de différentes expérimentations.

### (2) Traitement avec le dioxyde de carbone :

| Numérotation | | Activité résiduelle des différents facteurs de coagulation (%), après traitement au CO₂ supercritique en absence de stabilisant | | | | | |
|---|---|---|---|---|---|---|---|
| 911 MO 340 | | F V | F VII | F VIII | F IX | vWF | fibrinogène |
| 150 bars 0,5 h | 1.1 | 62 | 61 | 59 | 67 | 66 | 60 |
| 150 bars 1 h | 2.1 | 64 | 57 | 56 | 65 | 63 | 57 |
| 150 bars 1,5 h | 3.1 | 45 | 56 | 53 | 61 | 60 | 53 |
| 200 bars 0,5 h | 4.1 | 65 | 58 | 57 | 65 | 65 | 58 |
| 200 bars 1 h | 5.1 | 61 | 53 | 48 | 61 | 65 | 54 |
| 200 bars 1,5 h | 6.1 | 57 | 51 | 50 | 58 | 57 | 51 |
| 250 bars 0,5 h | 7.1 | 62 | 56 | 51 | 63 | 62 | 56 |
| 250 bars 1 h | 8.1 | 60 | 52 | 47 | 60 | 58 | 53 |
| 250 bars 1,5 h | 9.1 | 51 | 53 | 45 | 57 | 54 | 46 |

Le traitement au CO₂ en phase supercritique sur des plasmas non_stabilisés montre une perte d'activité biologique moyenne comprise entre 41 (0,5 h; 150 bars) et 55 % (1,5 h; 250 bars) des facteurs de coagulation sanguine.

Les rendements sont sensiblement inférieurs à ceux obtenus avec le protoxyde d'azote dans les mêmes conditions.

Ce phénomène est dû, en plus des causes évoquées pour le traitement au N₂O supercritique, à la baisse du pH provoquée par l'application sous pression du CO₂ en condition supercritique.

| Numérotation | | Activité résiduelle des différents facteurs de coagulation (%) après traitement au CO₂ supercritique en présence de FS1 | | | | | |
|---|---|---|---|---|---|---|---|
| 911 MO 341 | | F V | F VII | F VIII | F IX | vWF | fibrinogène |
| 150 bars 0,5 h | 10.1 | 87 | 65 | 87 | 80 | 95 | 85 |
| 150 bars 1 h | 11.1 | 86 | 85 | 87 | 88 | 85 | 70 |
| 150 bars 1,5 h | 12.1 | 83 | 85 | 82 | 86 | 90 | 81 |
| 200 bars 0,5 h | 13.1 | 86 | 84 | 85 | 90 | 86 | 83 |
| 200 bars 1 h | 14.1 | 82 | 50 | 70 | 80 | 95 | 84 |
| 200 bars 1,5 h | 15.1 | 80 | 79 | 79 | 85 | 80 | 79 |
| 250 bars 0,5 h | 16.1 | 83 | 75 | 81 | 88 | 84 | 82 |
| 250 bars 1 h | 17.1 | 80 | 80 | 81 | 84 | 82 | 78 |
| 250 bars 1,5 h | 18.1 | 77 | 77 | 78 | 81 | 84 | 85 |

Les rendements observés sur les préparations plasmatiques stabilisées avec la première formule de stabilisant montrent une amélioration très nette par rapport aux préparations plasmatiques non stabilisées.

La préservation de l'ordre de plus de 75 % de l'activité biologique des différents facteurs de coagulation pour des pressions de 250 bars avec des temps de contact de 1,5 heure démontre l'avantage de stabiliser le plasma ou ses dérivés par des agents stabilisants tels que les acides aminés.

Ces acides aminés, de par leurs propriétés stabilisantes, permettent de réduire de manière significative les problèmes mécanique et calorifique induits par le traitement.

| Numérotation | | Activité résiduelle des différents facteurs de coagulation (%) après traitement au CO₂ supercritique en présence de FS2 | | | | | |
|---|---|---|---|---|---|---|---|
| 911 MO 342 | | F V | F VII | F VIII | F IX | vWF | fibrinogène |
| 150 bars 0,5 h | 19.1 | 88 | 87 | 87 | 92 | 100 | 87 |
| 150 bars 1 h | 20.1 | 86 | 85 | 84 | 87 | 86 | 85 |
| 150 bars 1,5 h | 21.1 | 90 | 84 | 79 | 85 | 84 | 87 |
| 200 bars 0,5 h | 22.1 | 87 | 86 | 85 | 89 | 87 | 86 |
| 200 bars 1 h | 23.1 | 85 | 75 | 88 | 80 | 83 | 84 |
| 200 bars 1,5 h | 24.1 | 80 | 83 | 81 | 84 | 84 | 90 |
| 250 bars 0,5 h | 25 1 | 86 | 85 | 84 | 89 | 84 | 85 |
| 250 bars 1 h | 26.1 | 85 | 83 | 79 | 89 | 80 | 80 |
| 250 bars 1,5 h | 27.1 | 72 | 83 | 80 | 80 | 78 | 80 |

La seconde formule de stabilisant permet encore d'améliorer l'activité biologique des différentes protéines des plasmas traités au dioxyde de carbone en phase supercritique.

Les problèmes physicochimique et calorifique causés par le traitement sont fortement diminués par les solutions stabilisantes, et principalement par la seconde.

### II Etude d'intégrité des protéines plasmatiques après traitement au fluide supercritique.

L'éventuelle dénaturation des protéines du plasma est contrôlée par électrophorèse sur acétate de cellulose.

On n'observe aucune différence significative entre le plasma témoin non traité (911 MO 330 9.T) et les plasmas traités au N₂O supercritique (911 MO 330 9.1) et au CO₂ supercritique (911 MO 340 9.1); le profil électrophorétique ne révèle aucune dégradation protéique ni aucun phénomère d'agrégation.

Le tableau suivant indique les résultats observés sur les profils électrophorétiques :

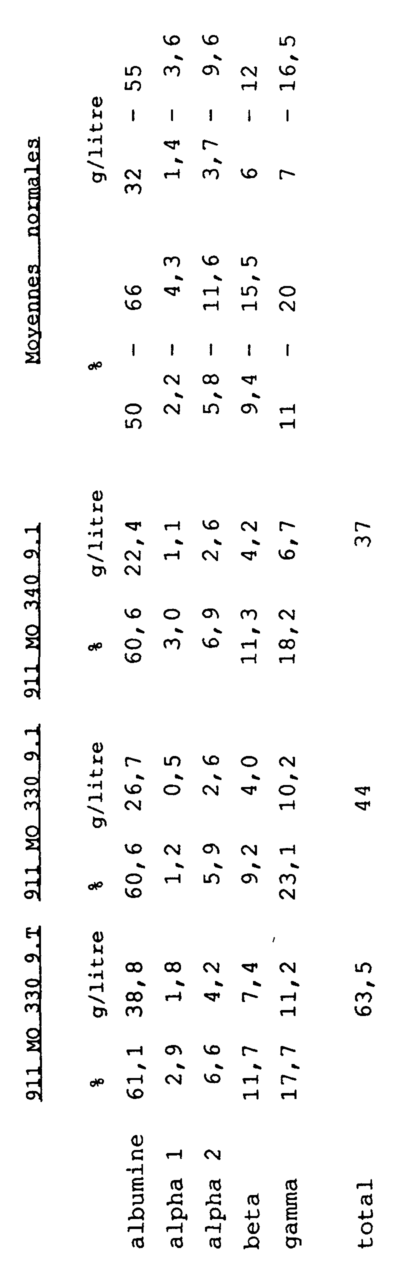

### III Mise en évidence de l'inactivation virale par le traitement au fluide supercritique - Première série d'essais

Dans ces essais, l'inactivation virale n'a été étudiée que sur les plasmas stabilisés avec la seconde formule de stabilisant (911 MO 332 et 911 MO 342), ces conditions étant les plus favorables pour la préservation de l'activité biologique des molécules étudiées (voir § I).

Les plasmas sont contaminés volontairement avec différents virus infectieux, titrant au départ de 5 à 7 log10. L'inactivation est mesurée aux temps t = 0,5 h, 1 h, 1,5 h pour le VIH-1 et au temps t = 1,5 h pour les autres virus.

### A. Inactivation virale par traitement au protoxyde d'azote supercritique à 37°C .

| Inactivation virale par traitement au N₂O supercritique | | |
|---|---|---|
| conditions de traitement : 250 bars durant 1,5 heure | | |
| Virus | Titre de l'inoculum (loq 10) | Titre résiduel |
| Sindbis | 5,8 | ND |
| Parainfluenza type 3 | 6,5 | ND |
| Herpès d'Aujesky | 6,2 | ND |
| Virus de la vaccine | 4,5 | ND |
| ND = non détectable | | |

| Inactivation virale du VIH-1 par traitement au N₂O supercritique à 250 bars | | |
|---|---|---|
| durée (en heures) | Titre de l'inoculum (loq 10) | Titre résiduel |
| 0,5 | 5,9 | ND |
| 1 | 5,9 | ND |
| 1,5 | 5,9 | ND |

### B. Inactivation virale par traitement au dioxyde de carbone supercritique à 32°C.

| Inactivation virale par traitement au CO₂ supercritique | | |
|---|---|---|
| conditions de traitement : 250 bars durant 1,5 heure | | |
| Virus | Titre de l'inoculum (loq 10) | Titre résiduel |
| Sindbis | 6,1 | ND |
| Parainfluenza type 3 | 5,5 | ND |
| Herpès d'Aujesky | 6,2 | ND |
| Virus de la vaccine | 5,0 | ND |
| ND = non détectable | | |

| Inactivation virale du VIH-1 par traitement au CO₂ supercritique | | |
|---|---|---|
| Durée (en heures) | Titre de l'inoculum (log 10) | Titre résiduel |
| 0,5 | 5,5 | ND |
| 1 | 5,5 | ND |
| 1,5 | 5,5 | ND |

Ces résultats indiquent que les traitements au protoxyde d'azote et au dioxyde de carbone en phase supercritique permettent d'inactiver rapidement des doses élevées de virus dans les conditions définies plus haut, tout en permettant de préserver l'activité biologique des protéines plasmatiques.

### IV Mise en évidence de l'inactivation virale par le traitement au fluide supercritique - Deuxième série d'essais

Chaque mode de traitement par le N₂O supercritique est expérimenté séparément et dans deux expériences indépendantes avec des préparations présentant des titres élevés en virus des espèces suivantes, polio Sabin 1 (virus à ARN non enveloppé), Reovirus de type 3 (virus à ARN non enveloppé), virus Sindbis (virus à ARN enveloppé) et virus de la pseudorage (virus à ADN enveloppé). Un échantillon est prélevé pour déterminer la dose virale initiale.

Dans une première étape, un échantillon de plasma humain contaminé est traité à 50°C sous 250 bars de pression durant 2 heures en présence de 1300 g de sorbitol, 514 g de saccharose, 4mM de gluconate de calcium, 15mM de citrate trisodique, 5 g de lysine et 5 g d'arginine par litre de plasma initial. Dans une deuxième série d'expériences le plasma est traité à 37°C sous 250 bars de pression durant 2 heures en présence de 4mM de gluconate de calcium, 15mM de citrate trisodique, 5 g de lysine et 5g d'arginine par litre de plasma initial. Pendant la mise en oeuvre de l'inactivation par les fluides supercritiques, des échantillons sont prélevés à des moments prédéterminés (0,5 h, 1h, 1,5h et 2 h) et testés pour déterminer l'infectiosité virale résiduelle. Les valeurs trouvées sont utilisées pour déterminer la cinétique d'inactivation virale.

Les facteurs de réduction sont calculés pour chaque étape en comparant le titre de virus restant après le traitement, par rapport à la dose virale initiale.

Les virus choisis représentent une sélection de virus modèles présentant des résistances variables aux traitements thermiques. En particulier, le virus Sindbis est utilisé comme modèle pour le virus de l'hépatite C en raison de l'inaptitude à propager et tester ce virus. Le virus de la pseudorage présente une résistance proche de celle du virus de l'hépatite B.

Le virus polio Sabin 1 est un virus à ARN simple brin, non enveloppé, qui est considéré comme un modèle pour le virus de l'hépatite A. Il est en général sensible aux solvants organiques, stable aux pH acides et est facilement inactivé par la chaleur. Ce virus a été obtenu auprès du Laboratoire Vétérinaire Central (Central Veterinary Laboratory), Surrey, (Royaume-Uni).

Le Reovirus de type 3 est un virus à ARN double brin, non enveloppé. Il est résistant aux solvants organiques, stable aux pH acides et sensible aux pH alcalins. Ce virus a été obtenu de l'American Type Culture Collection (ATCC), Maryland, EUA.

Le virus Sindbis est un virus à ARN simple brin, enveloppé et est-accepté par les instances réglementaires comme modèle pour le virus de l'hépatite C. Il est sensible aux solvants des lipides et relativement thermostable. Ce virus a été obtenu auprès du Laboratoire Vétérinaire Central cité plus haut.

Le virus de la pseudorage est un virus à ADN double brin, enveloppé. Il est relativement thermostable, sensible aux solvants des lipides et aux valeurs extrêmes de pH. Ce virus a été obtenu auprès de l'ATCC citée plus haut.

Des lots supplémentaires sont préparés comme témoins et titrés en même temps que les échantillons traités (les échantillons témoins sont traités de la même manière que les échantillons soumis à l'inactivation virale ; par exemple, des étapes d'ultracentrifugation, de filtration stérilisante et de congélation-décongélation sont inclues, si nécessaire).

### Préparation des stocks de virus

### 1. Virus polio Sabin 1 :

Des monocouches de cellules MRC-5 sont préparées, infectées par le virus, puis incubées jusqu'à observation d'un effet cytopathologique avancé. Les monocouches infectées sont rompues de façon aseptique par congélation-décongélation et centrifugées puis le surnageant est récolté. La lignée cellulaire MRC-5 a été obtenue auprès de l'European Cell Culture Collection, Wiltshire (Royaume-Uni).

### 2. Reovirus de type 3 :

Des monocouches de cellules LLC-MK₂ (rein de singe) sont préparées et infectées par le virus jusqu'à observation d'un effet cytopathologique avancé. Les monocouches infectées sont rompues de façon aseptique par congélation-décongélation et centrifugées puis le surnageant est récolté. La lignée cellulaire LLC-MK₂ a été obtenue auprès des Laboratoires Flow (Flow Laboratories), Ayrshire, (Royaume-Uni).

### 3. Virus Sindbis :

Des monocouches de cellules Vero sont préparées et infectées par le virus jusqu'à observation d'un effet cytopathologique avancé. Les monocouches infectées sont rompues de façon aseptique par congélation-décongélation et centrifugées puis le surnageant est récolté. La lignée cellulaire Vero a été obtenue auprès de l'European Cell Culture Collection citée plus haut.

### 4. Virus de la pseudorage

Des monocouches de cellules Vero sont préparées et infectées par le virus jusqu'à observation d'un effet cytopathologique avancé. Les monocouches infectées sont rompues de façon aseptique par congélation-décongélation et centrifugées puis le surnageant est récolté. La lignée cellulaire Véro a été obtenue auprès de l'European Cell Culture Collection citée plus haut.

### Titrage des virus :

Les titres du virus polio Sabin 1, du Reovirus de type 3 et du virus de la pseudorage sont déterminés par mesure de la TCID₅₀. Le titre du virus Sindbis est déterminé par dosage par plages.

### Mesure de la TCID₅₀

Des plaques pour culture de cellules stériles sont ensemencées pour donner des monocouches confluentes de cellules. Les échantillons d'essai sont dilués en série au 1/₁₀e, si cela est approprié, dans du milieu de maintenance pour cellules.

Des témoins positifs d'échantillons des stocks de virus utilisés pour l'expérience sont inclus dans les étapes de congélation-décongélation et les étapes d'ultra-centrifugation, quand cela est nécessaire, pour contrôler toute diminution possible de l'infectiosité virale.

Le milieu est éliminé des plaques et 0,5 ml de l'échantillon d'essai ou de la-dilution de virus témoin approprié est ajouté à chacun des 8 puits de la plaque utilisée. Des séries de puits de témoins négatifs sont inclues en utilisant du milieu de maintenance pour cellules comme inoculum. Les plaques sont ensuite incubées à 37°C pendant 90 minutes dans une atmosphère humide contenant 5% de dioxyde de carbone, après quoi les inoculums sont éliminés. Les couches de cellules inoculées avec les dilutions inférieures d'échantillon sont lavées avec une solution saline tamponnée au phosphate puis du milieu de maintenance pour cellules est ajouté à chaque puits. Les plaques sont ensuite incubées à 37°C en faisant des observations régulières de l'effet cytopathologique du virus pendant une durée allant jusqu'à 14 jours.

### Dosage par plages :

Des dilutions au 1/₁₀e des échantillons d'essai et de témoins positifs sont réalisées, lorsque cela est approprié. Chaque dilution de virus est inoculée sur 4 puits parallèles contenant des monocouches confluentes de cellules détectrices.

Après élimination du milieu de croissance des monocouches de cellules, on ajoute 0,5 ml de chaque dilution de virus, on l'adsorbe pendant 90 min à 37°C puis on l'élimine. Les monocouches de cellules sont alors lavées une fois avec une solution saline tamponnée au phosphate avant d'être recouvertes avec du milieu contenant 2% de sérum de veau foetal et 1,5% de carboxyméthylcellulose.

Les plaques sont incubées, sans les déranger, pendant une durée de 3 jours puis sont fixées et colorées, et on effectue le comptage des plages.

### Essais d'inactivation virale :

On utilise une installation pilote de traitement par les fluides supercritiques dans les conditions indiquées plus haut (50°C ; 250 bars ; ou 37°C ; 250 bars).

Des fractions de 75 ml de plasma humain sont contaminées artificiellement, en double, avec 1,0 ml de solutions de virus titrant environ 10⁷ unités TCID₅₀, du virus polio Sabin 1, du Reovirus de type 3 ou du virus de la pseudorage, et environ 10 ufp du virus Sindbis. Après mélange, on prélève des aliquotes de 1,0 ml pour déterminer le titre viral initial.

### Analyse des résultats :

Un facteur de réduction pour chaque virus à chaque température est calculé, qui représente la moyenne des 2 valeurs. Le facteur de réduction est déterminé comme étant la réduction du titre du virus obtenue à la suite du traitement, par comparaison avec le titre viral initial.

Les résultats obtenus sont rassemblés dans les tableaux qui suivent.

| Inactivation virale par traitement au N₂O supercritique | | |
|---|---|---|
| Conditions de traitement : 250 bars durant 2 heures à 50°C | | |
| Virus | Titre de l'inoculum (log₁₀) | Facteur de réduction (log₁₀) |
| Virus polio Sabin 1 | 6,4 | > 5,4 |
| Reovirus de type 3 | 5,5 | >4,8 |
| Virus de la pseudorage | 6,2 | >4,9 |
| Virus Sindbis | 6,5 | >5,5 |

| Inactivation virale par traitement au N₂O supercritique | | |
|---|---|---|
| Conditions de traitement : 250 bars durant 2 heures à 37°C | | |
| Virus | Titre de l'inoculum (log₁₀) | Facteur de réduction (log₁₀) |
| Virus polio Sabin 1 | 6,4 | >5,4 |
| Reovirus de type 3 | 5,5 | >4,5 |
| Virus de la pseudorage | 6,2 | >4,9 |
| Virus Sindbis | 6,5 | >5,5 |

### V Mise en évidence d'une délipidation du plasma par le traitement au fluide supercritique

Le traitement par les fluides supercritiques permet l'élimination de plus de 70 % de lipides comme les triglycérides et le cholestérol contenus dans les plasmas humains et animaux. Les lipides extraits sont récupérés dans les séparateurs.

Les résultats de délipidation ne sont donnés que pour les plasmas 911 MO 332 et 911 MO 342, c'est-à-dire pour les plasmas stabilisés avec la seconde formule de stabilisants. Les résultats de délipidation sont identiques pour les sous-lots 911 MO 330, 911 MO 331, 911 MO 340 et 911 MO 341.

Les rendements sont établis par rapport à un témoin non traité, durant le temps de manipulation à 37°C.

| Pourcentage de délipidation du plasma avec le protoxyde d'azote supercritique | | |
|---|---|---|
| 911 MO 332 | triglycérides (%) | cholestérol (%) |
| 19.1 (150 bars; 0,5 h) | 43 | 47 |
| 20.1 (150 bars; 1 h) | 51 | 50 |
| 21.1 (150 bars; 1,5 h) | 54 | 59 |
| 22.1 (200 bars; 0,5 h) | 50 | 53 |
| 23.1 (200 bars; 1 h) | 55 | 58 |
| 24.1 (200 bars; 1,5 h) | 62 | 62 |
| 25.1 (250 bars; 0,5 h) | 48 | 55 |
| 26.1 (250 bars; 1 h) | 61 | 63 |
| 27.1 (250 bars; 1,5 h) | 63 | 71 |

| Pourcentage de délipidation du plasma avec le protoxyde d'azote supercritique | | |
|---|---|---|
| 911 MO 342 | triglycérides (%) | cholestérol (%) |
| 19.1 (150 bars; 0,5 h) | 45 | 46 |
| 20.1 (150 bars; 1 h) | 56 | 53 |
| 21.1 (150 bars; 1,5 h) | 60 | 63 |
| 22.1 (200 bars; 0,5 h) | 50 | 51 |
| 23.1 (200 bars; 1 h) | 53 | 59 |
| 24.1 (200 bars; 1,5 h) | 66 | 68 |
| 25.1 (250 bars; 0,5 h) | 46 | 59 |
| 26.1 (250 bars; 1 h) | 63 | 66 |
| 27.1 (250 bars; 1,5 h) | 72 | 76 |

De plus, l'analyse d'un lipoprotéinogramme [kit Lipofilm (Sebia), 25 volts, 45 minutes] des fractions de plasma traitées au protoxyde d'azote et au dioxyde de carbone, à 250 bars et 37°C pendant 1 h 30 (911 MO 332 27.1 et 911 MO 342 27.1) et d'un témoin maintenu à 37°C pendant 1 h 30 montre clairement la disparition de la bande correspondant aux HDL et la diminution des bandes correspondant aux LDL et aux VLDL, et l'augmentation importante de la bande située au dépôt, ce qui résulte de la dénaturation des lipides au cours du traitement. (L'expérience ayant été réalisée en conditions de mise en contact statique comme définie ci-dessus, les molécules dénaturées restent présentes sur le lipidogramme).

## Revendications

1. Procédé d'inactivation virale des produits plasmatiques, **caractérisé en ce qu'**il comporte une mise en contact desdits produits avec un fluide constitué par un corps pur ou un mélange de corps purs en état supercritique ou subcritique, choisi parmi les hydrocarbures en C₂ à C₈, de préférence en C₂ à C₆, les hydrocarbures halogénés, le dioxyde de carbone, le protoxyde d'azote et leurs mélanges.

2. Procédé d'inactivation virale des produits plasmatiques selon la revendication 1, **caractérisé en ce qu'**il est mis en oeuvre en utilisant, comme fluide de traitement, le dioxyde de carbone ou le protoxyde d'azote en état supercritique ou subcritique, à une température comprise entre 10 et 65°C et sous une pression comprise entre Pc et 10 Pc, Pc étant la pression critique du fluide utilisé, pendant tout ou partie du traitement.

3. Procédé d'inactivation virale des produits plasmatiques selon la revendication 2, **caractérisé en ce que** la température de traitement est comprise entre 30 et 65°C, pendant tout ou partie du traitement.

4. Procédé d'inactivation virale des produits plasmatiques selon la revendication 2 ou 3, **caractérisé en ce que** la pression opératoire est comprise entre P_{c} et 5 P_{c}, pendant tout ou partie du traitement.

5. Procédé d'inactivation virale des produits plasmatiques selon l'une quelconque des revendications 1 à 4, **caractérisé en ce que** le fluide est choisi parmi les corps ou mélanges de corps ne modifiant pas le pH desdits produits pendant leur traitement.

6. Procédé d'inactivation virale des produits plasmatiques selon l'une quelconque des revendications 1 à 5, **caractérisé en ce que** les produits plasmatiques sont additionnés de stabilisants des protéines qu'ils contiennent, notamment lorsque les températures de traitement sont supérieures à 40°C.

7. Procédé d'inactivation virale des produits plasmatiques selon la revendication 6, **caractérisé en ce que** lesdits stabilisants sont des sucres, des polyols ou des acides aminés, seuls ou en combinaison.

8. Procédé d'inactivation virale des produits plasmatiques selon la revendication 7, **caractérisé en ce que** lesdits stabilisants sont choisis dans le groupe constitué par le saccharose, le glucose, le fructose, le sorbitol, le mannitol, le glycérol, le glycocolle, l'arginine et la lysine.

9. Procédé d'inactivation virale des produits plasmatiques selon la revendication 7 ou 8, **caractérisé en ce que** les produits plasmatiques sont en outre additionnés d'au moins un autre stabilisant choisi parmi le chlorure de calcium, le gluconate de calcium et l'héparine.

10. Procédé d'inactivation virale des produits plasmatiques selon l'une quelconque des revendications 1 à 9, **caractérisé en ce que** le fluide est additionné d'un ou plusieurs corps choisis parmi les hydrocarbures en C₉ ou plus, les composés organiques oxygénés et les composés organiques azotés.

11. Procédé d'inactivation virale des produits plasmatiques selon l'une quelconque des revendications 1 à 10 et de délipidation de ces produits, **caractérisé en ce que** le fluide en état supercritique ou subcritique présente un caractère lipophile.

## Patentansprüche

1. Verfahren zur Virusinaktivierung von Plasmaprodukten, **dadurch gekennzeichnet, dass** es das Inkontaktbringen dieser Produkte mit einem Fluid umfasst, das aus einem reinen Stoff oder einem Gemisch von reinen Stoffen in überkritischem oder unterkritischem Zustand, ausgewählt aus C₂ bis C₈-Kohlenwasserstoffen, vorzugsweise C₂ bis C₆-Kohlenwasserstoffen, Halogenkohlenwasserstoffen, Kohlendioxid, Distickstoffoxid und ihren Gemischen, gebildet ist.

2. Verfahren zur Virusinaktivierung von Plasmaprodukten nach Anspruch 1, **dadurch gekennzeichnet, dass** es unter Verwendung von Kohlendioxid oder Distickstoffoxid in überkritischem oder unterkritischem Zustand als Behandlungsfluid bei einer Temperatur zwischen 10 und 65°C und unter einem Druck zwischen Pc und 10 Pc, wobei Pc der kritische Druck des verwendeten Fluids ist, während der gesamten Behandlung oder eines Teils davon durchgeführt wird.

3. Verfahren zur Virusinaktivierung von Plasmaprodukten nach Anspruch 2, **dadurch gekennzeichnet, dass** die Behandlungstemperatur während der gesamten Behandlung oder eines Teils davon zwischen 30 und 65°C liegt.

4. Verfahren zur Virusinaktivierung von Plasmaprodukten nach Anspruch 2 oder 3, **dadurch gekennzeichnet, dass** der Betriebsdruck während der gesamten Behandlung oder eines Teils davon zwischen Pc und 5 Pc liegt.

5. Verfahren zur Virusinaktivierung von Plasmaprodukten nach einem der Ansprüche 1 bis 4, **dadurch gekennzeichnet, dass** das Fluid ausgewählt wird aus Stoffen oder Stoffgemischen, die den pH dieser Produkte während ihrer Behandlung nicht verändern.

6. Verfahren zur Virusinaktivierung von Plasmaprodukten nach einem der Ansprüche 1 bis 5, **dadurch gekennzeichnet, dass** die Plasmaprodukte mit Stabilisatoren der Proteine, welche sie enthalten, versetzt werden, insbesondere, wenn die Temperaturen der Behandlung höher als 40°C sind.

7. Verfahren zur Virusinaktivierung von Plasmaprodukten nach Anspruch 6, **dadurch gekennzeichnet, dass** die Stabilisatoren Zucker, Polyole oder Aminosäuren, allein oder in Kombination sind.

8. Verfahren zur Virusinaktivierung von Plasmaprodukten nach Anspruch 7, **dadurch gekennzeichnet, dass** die Stabilisatoren aus der von Saccharose, Glucose, Fructose, Sorbitol, Mannitol, Glycerin, Glycin, Arginin und Lysin gebildeten Gruppe ausgewählt werden.

9. Verfahren zur Virusinaktivierung von Plasmaprodukten nach Anspruch 7 oder 8, **dadurch gekennzeichnet, dass** die Plasmaprodukte außerdem mit mindestens einem weiteren Stabilisator, ausgewählt aus Calciumchlorid, Calciumgluconat und Heparin, versetzt werden.

10. Verfahren zur Virusinaktivierung von Plasmaprodukten nach einem der Ansprüche 1 bis 9, **dadurch gekennzeichnet, dass** das Fluid mit einem oder mehreren Stoffen, ausgewählt aus Kohlenwasserstoffen mit 9 oder mehr Kohlenstoffatomen, sauerstoffhaltigen organischen Verbindungen und stickstoffhaltigen organischen Verbindungen, versetzt wird.

11. Verfahren zur Virusinaktivierung von Plasmaprodukten nach einem der Ansprüche 1 bis 10 und zur Entfemung der Lipide aus diesen Produkten, **dadurch gekennzeichnet, dass** das Fluid in überkritischem oder unterkritischem Zustand einen lipophilen Charakter aufweist.

## Claims

1. A method for the viral inactivation of plasmatic products, wherein said method includes bringing into contact said products with a fluid consisting of an elementary body or a mixture of elementary bodies in a supercritical or subcritical state, selected from hydrocarbons of C₂-C₈, preferably of C₂-C₆, halogenated hydrocarbons, carbon dioxide, nitrogen monoxide and mixtures thereof.

2. A method for the viral inactivation of plasmatic products according to claim 1, wherein said method is carried out using carbon dioxide or nitrogen monoxide as treatment fluid in a supercritical or subcritical state, at a temperaure of from 10 to 65°C and at a pression of from Pc to 10 Pc, Pc being the critical pression of the fluid used during the whole or a part of the treatment.

3. A method for the viral inactivation of plasmatic products according to claim 2, wherein the treatment temperature is of from 30 to 65°C during the whole or a part of the treatment.

4. A method for the viral inactivation of plasmatic products according to claim 2 or 3, wherein the working pression is of from Pc to 5 Pc, during the whole or a part of the treatment.

5. A method for the viral inactivation of plasmatic products according to any of claims 1 to 4, wherein the fluid is selected from bodies or mixture of bodies not modifying the pH of said products during their treatment.

6. A method for the viral inactivation of plasmatic products according to any of claims 1 to 5, wherein the plasmatic products are supplemented with stabilizers of their constitutive proteins, in particular when temperatures are higher than 40°C.

7. A method for the viral inactivation of plasmatic products according to claim 6, wherein said stabilizers are sugars, polyalcohols or amino acids, alone or in combination.

8. A method for the viral inactivation of plasmatic products according to claim 7, wherein said stabilizers are selected from the group consisting of sucrose, glucose, fructose, sorbitol, mannitol, glycerol, glycocolle, arginine and lysine.

9. A method for the viral inactivation of plasmatic products according to claim 7 or 8, wherein at least one other stabilizer selected from calcium chloride, calcium gluconate and heparin is further added to the plasmatic products.

10. A method for the viral inactivation of plasmatic products according to any of claims 1 to 9, wherein one or more bodies selected from hydrocarbons of C₉ or more, oxygenated organic compounds and nitrogenous organic compounds are added to the fluid.

11. A method for the viral inactivation of plasmatic products according to any of claims 1 to 10 and for delipidation of said compounds, wherein the fluid in a supercritical or subcritical state presents a lipophilic characteristic.
